(19) Europäisches Patentamt
European Patent Office
Office européen des brevets

(11) EP 0 647 136 B1

(12) EUROPÄISCHE PATENTSCHRIFT

(45) Veröffentlichungstag und Bekanntmachung des Hinweises auf die Patenterteilung:
**19.03.1997 Patentblatt 1997/12**

(21) Anmeldenummer: **93914664.3**

(22) Anmeldetag: **16.06.1993**

(51) Int. Cl.$^6$: **A61K 31/44**, A61K 31/47

(86) Internationale Anmeldenummer:
**PCT/EP93/01531**

(87) Internationale Veröffentlichungsnummer:
**WO 94/00122 (06.01.1994 Gazette 1994/02)**

(54) **VERWENDUNG VON KONDENSIERTE BIS-(3,4-DIHYDRO-PYRIDINYL)METHANE ZUR BEHANDLUNG VON MORBUS CROHN, COLITIS ULCEROSA, CHRONISCH INFLAMMATORISCHEN PROZESSEN UND ALS ANTIPROLIFERATIVE MITTELN**

USE OF CONDENSED BIS-(3,4-DIHYDRO-PYRIDINYL)METHANE FOR TREATING CROHN'S DISEASE, COLITIS ULCEROSA, CHRONIC INFLAMATORY PROCESSES AND AS AN ANTIPROLIFERATIVE

UTILISATION DE BIS-(3,4-DIHYDRO-PYRIDINYL)METHANE CONDENSE DANS LE TRAITEMENT DE LA MALADIE DE CROHN, DE LA COLITE ULCEREUSE, DE PROCESSUS INFLAMMATOIRES CHRONIQUES ET EN TANT QU'AGENT ANTIPROLIFERATIF

(84) Benannte Vertragsstaaten:
**AT BE CH DE DK ES FR GB GR IE IT LI LU MC NL PT SE**

(30) Priorität: **22.06.1992 DE 4220324**
**22.06.1992 DE 4220320**
**22.06.1992 DE 4220379**

(43) Veröffentlichungstag der Anmeldung:
**12.04.1995 Patentblatt 1995/15**

(73) Patentinhaber:
- **BOEHRINGER INGELHEIM INTERNATIONAL GmbH**
  **D-55216 Ingelheim am Rhein (DE)**
  Benannte Vertragsstaaten:
  **GB IE**
- **BOEHRINGER INGELHEIM KG**
  **55216 Ingelheim (DE)**
  Benannte Vertragsstaaten:
  **BE CH DE DK ES FR GR IT LI LU MC NL PT SE AT**

(72) Erfinder:
- **ARNDTS, Dietrich**
  **D-55437 Appenheim (DE)**
- **LÖSEL, Walter**
  **D-6535 Gau-Algesheim (DE)**
- **ROOS, Otto**
  **D-6501 Schwabenheim (DE)**

(56) Entgegenhaltungen:
**EP-A- 0 288 048** **EP-A- 0 404 525**

- **PFLÜGERS ARCHIV EUROPEAN JOURNAL OF PHYSIOLOGY, Bd. 420, Nr. 3-4, M rz 1992, Seiten 319-328; SIEMER, CHRISTIANE ET AL.: 'ACTIVATION OF NONSELECTIVE CATION CHANNELS IN THE BASOLATERAL MEMBRANE OF RAT DISTAL COLON CRYPT CELLS BY PROSTAGLANDIN E2'**
- **THE LANCET, Bd. 339, Nr. 8790, 15. Februar 1992, Seiten 381-385; MURCH, S.H. ET AL.: 'HIGH ENDOTHELIN-1 IMMUNOREACTIVITY IN CROHN DISEASE AND ULCERATIVE COLITIS'**
- **CLINICAL AND INVESTIGATIVE MEDICINE, Bd. 14, Nr. 6, Dezember 1991, Seiten 499-507; SIMONSON, M.S. ET AL.: 'THE EFFECTS OF ENDOTHELIN ON CYTOSOLIC CALCIUM IN CULTURED HUMAN AND RAT GLOMERULAR MESANGIAL CELLS'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.17/3.4

## Beschreibung

Die Erfindung betrifft kondensierte Bis-(3,4-dihydro-1-pyridinyl)methane und deren Verwendung für die Herstellung von pharmazeutischen Zubereitungen für die Behandlung chronisch inflammatorischer Prozesse, von Colitis Ulcerosa und des Morbus Crohn und von pharmazeutischen Zubereitungen mit antiproliferativer Wirkung.

Die genannten Verbindungen haben die allgemeine Formel I

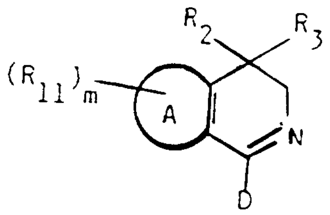

I

worin

A einen Benzo- oder Thienorest bedeutet;

$R_2$ und $R_3$ unabhängig voneinander Wasserstoff oder $(C_1\text{-}C_5)$Alkyl oder gemeinsam mit dem Kohlenstoffatom, an das sie gebunden sind, einen 5- oder 6-gliedrigen Carbocyclus bedeuten;

$R_{11}$ $(C_1\text{-}C_4)$Alkyl, Halogen (F, Cl, Br, J), Hydroxy, $(C_1\text{-}C_4)$Alkoxy, Amino, Thiomethyl, Methansulfonyloxy oder Methansulfonamido bedeutet, oder zwei benachbarte Substituenten $R_{11}$ zusammen $-O-CH_2-O-$ oder $-O-CH_2-CH_2-O-$ sind;

m 0, 1, 2 oder 3 bedeutet, wenn A ein Benzorest ist, und 0, 1 oder 2 bedeutet, wenn A ein Thienorest ist;

und D eine Gruppe der Formel Ia bedeutet

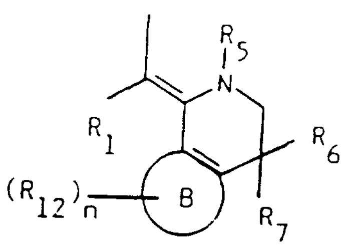

Ia

wobei in der Gruppe der Formel Ia

B einen Benzo- oder Thienorest bedeutet;

$R_1$ Wasserstoff, $(C_1\text{-}C_{10})$Alkyl, Phenyl, Phenyl$(C_1\text{-}C_5)$alkyl, $(C_1\text{-}C_4)$Alkoxy oder -NHCOX (worin X $(C_1\text{-}C_5)$Alkyl ist) bedeutet;

$R_5$ Wasserstoff, $(C_1\text{-}C_4)$Alkyl oder Hydroxymethyl bedeutet;

$R_6$ und $R_7$ unabhängig voneinander Wasserstoff oder $(C_1\text{-}C_5)$Alkyl oder gemeinsam mit dem Kohlenstoffatom, an das sie gebunden sind, einen 5- oder 6-gliedrigen Carbocylcus bedeuten;

$R_{12}$ $(C_1\text{-}C_4)$Alkyl, Halogen (F, Cl, Br, J), Hydroxy, $(C_1\text{-}C_4)$Alkoxy, Amino, Thiomethyl, Methansulfonyloxy oder Methansulfonamido bedeutet, oder zwei benachbarte Substituenten $R_{12}$ zusammen $-O-CH_2-O-$ oder $-O-CH_2-CH_2-O-$ sind;

und n 0, 1, 2 oder 3 bedeutet, wenn B ein Benzorest ist, und 0, 1 oder 2 bedeutet, wenn B ein Thienorest ist;

und ihre pharmazeutisch annehmbaren Salze mit anorganischen oder organischen Säuren.

Unter dem Carbocyclus, der von $R_2$ und $R_3$ beziehungsweise $R_6$ und $R_7$ und dem jeweiligen Kohlenstoffatom, an das sie gebunden sind, gebildet wird, ist vorzugsweise ein gesättigter 5- oder 6-gliedriger Carbocyclus zu verstehen.

Verbindungen der Formel I, worin D die Gruppe der Formel Ia ist, werden im folgenden als Verbindungen der Formel VIII bezeichnet.

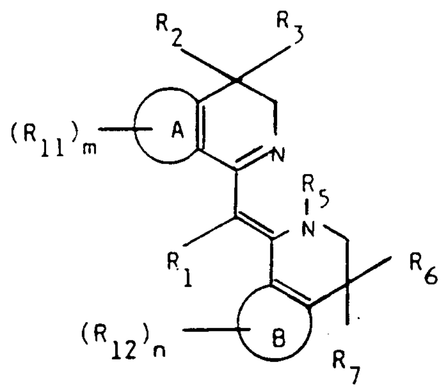

VIII

Verbindungen der Formel VIII, worin $R_5$ Wasserstoff ist, bilden Tautomere der Formeln VIIIa und VIIIb,

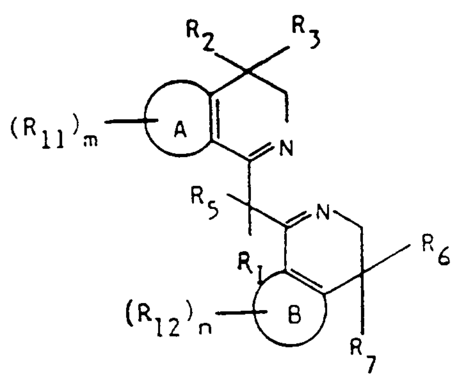

VIIIa

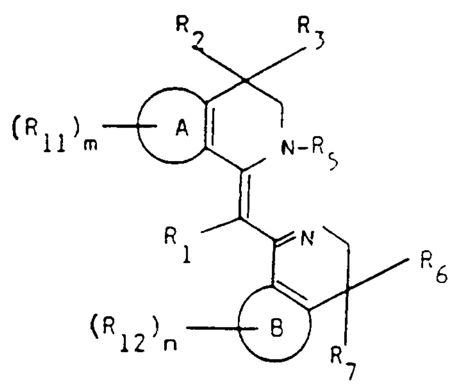

VIIIb

worin $R_5$ ebenfalls Wasserstoff ist. Die Definition der Formel I bzw. VIII umfaßt auch die genannten Tautomeren.

Von Kobor, Jeno wurde in Szegedi Tonarkepzo Foiskola Tud. Kozl. 1975, Seiten 145-153 (vgl. Chem. Abstr. 87; 13498OZ) die Verbindung der allgemeinen Formel VIII

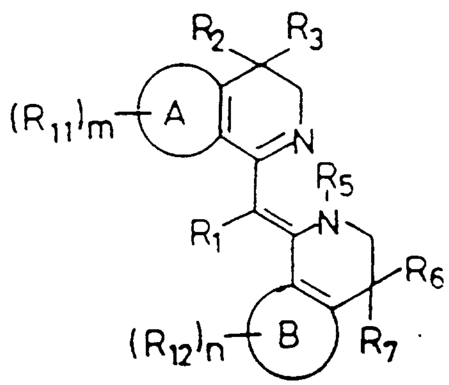

VIII

und ihre Herstellung beschrieben, worin $R_1$, $R_2$, $R_3$, $R_5$, $R_6$ und $R_7$ Wasserstoff bedeuten und die substituierten Gruppen A und B den Rest

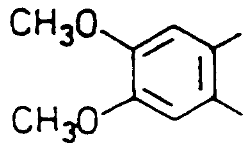

bedeuten. Diese Publikation enthält keine Angaben über physiologische Wirkungen dieser Verbindung. In der europäischen Patentanmeldung 288 048 sind die Verbindungen der allgemeinen Formel I als Verbindungen mit cardio- und/oder hirnprotektiver Wirkung beschrieben worden.

Von den Verbindungen der Formel VIII können erfindungsgemäß folgende als besonders interessant bevorgehoben werden:

-) Verbindungen VIII, worin $R_1$ Wasserstoff, $(C_1\text{-}C_{10})$Alkyl, Phenyl$(C_1\text{-}C_5)$alkyl oder -NHCOX (worin X $(C_1\text{-}C_5)$Alkyl ist) bedeutet;
$R_{11}$ und $R_{12}$ unabhängig voneinander $(C_1\text{-}C_4)$Alkyl, Hydroxy, $(C_1\text{-}C_4)$Alkoxy, Methansulfonyloxy oder Methansulfonamido bedeuten, oder zwei benachbarte Substituenten $R_{11}$ beziehungsweise $R_{12}$ zusammen $-O-CH_2-O-$ oder $-O-CH_2-CH_2-O-$ sind;

-) Verbindungen VIII, worin $R_1$ Wasserstoff, $(C_1\text{-}C_{10})$Alkyl oder -NHCOX (worin X $(C_1\text{-}C_5)$Alkyl ist) bedeutet;
$R_2$, $R_3$, $R_6$ und $R_7$ unabhängig von einander Wasserstoff bedeuten oder $R_2$ mit $R_3$ und/oder $R_6$ mit $R_7$ und dem jeweiligen Kohlenstoffatom, an das sie gebunden sind, einen 5- oder 6-gliedrigen Carbocyclus bedeuten;
$R_{11}$ und $R_{12}$ unabhängig voneinander Hydroxy, $(C_1\text{-}C_4)$Alkoxy, Methansulfonyloxy oder Methansulfonamido bedeuten, oder zwei benachbarte Substituenten $R_{11}$ beziehungsweise $R_{12}$ zusammen $-O-CH_2-O-$ oder $-O-CH_2-CH_2-O-$ sind;

-) insbesondere Verbindungen, worin $R_1$ Wasserstoff, $(C_1\text{-}C_6)$Alkyl, oder $-NHCOCH_3$ bedeutet und/oder
$R_5$ Wasserstoff, Methyl oder Hydroxymethyl bedeutet und/oder
$R_2$, $R_3$, $R_6$ und $R_7$ unabhängig voneinander Wasserstoff bedeuten oder $R_2$ mit $R_3$ und/oder $R_6$ mit $R_7$ und dem jeweiligen Kohlenstoffatom, an das sie gebunden sind, einen 5-gliedrigen Carbocyclus bedeuten und/oder
$R_{11}$ und $R_{12}$ unabhängig voneinander Hydroxy, Methoxy, Methansulfonyloxy oder Methansulfonamido bedeuten, oder zwei benachbarte Substituenten $R_{11}$ beziehungsweise $R_{12}$ zusammen $-O-CH_2-O-$ sind.

Besonders hervorzuheben sind Verbindungen

-) worin $R_1$ und $R_5$ Wasserstoff sind, und/oder worin $R_2$, $R_3$, $R_6$ und $R_7$ Wasserstoff sind oder worin $R_2$ und $R_3$ beziehungsweise $R_6$ und $R_7$ je zusammen $-(CH_2)_4-$ sind; insbesondere Verbindungen, worin A und B unabhängig voneinander je eine Thienogruppe sind, vorzugsweise 2,3-Thieno, vorzugsweise unsubstituierte Thienogruppe sind

beziehungsweise Verbindungen

-) worin A und/oder B ein Benzorest ist, worin m beziehungsweise n 2 bedeuten, wobei vorzugsweise die beiden Substituenten $R_{11}$ und/oder $R_{12}$ im Benzorest in meta- beziehungsweise para-Position zu den Fusionspunkten des Restes A beziehungsweise B stehen und vorzugsweise diese Substituenten $R_{11}$ und $R_{12}$ unabhängig voneinander Hydroxy, $(C_1\text{-}C_4)$Alkoxy (vorzugsweise Methoxy) oder die 2 benachbarten Substituenten $R_{11}$ beziehungsweise $R_{12}$ zusammen $-0-CH_2-CH_2-0-$ oder $-0-CH_2-0-$ sind.

Hervorzuheben sind Verbindungen, worin $R_{11}$ und $R_{12}$ Methoxy sind.

Beispiele wirksamer Verbindungen gemäß der Erfindung sind in den Tabellen weiter unten angeführt, hervorzuheben sind

Verbindung A

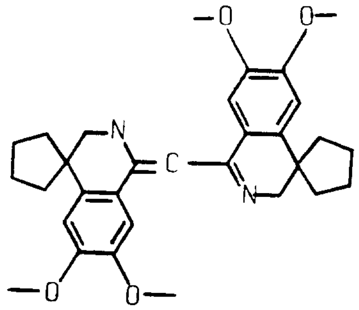

Verbindung B

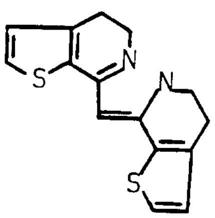

Verbindung C

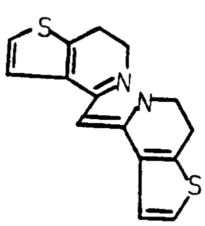

Verbindung D

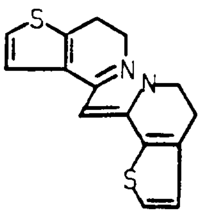

und deren pharmazeutisch annehmbare Salze.

Die Verbindungen können nach an sich bekannten Verfahren (z.B. gemäß EP 288 048) hergestellt werden.

Zur Salzbildung geeignete Säuren sind beispielsweise Salzsäure, Bromwasserstoffsäure, Schwefelsäure, Phosphorsäure, Salpetersäure, Essigsäure, Propionsäure, Buttersäure, Oxalsäure, Malonsäure, Bernsteinsäure, Maleinsäure, Fumarsäure, Milchsäure, Weinsäure, Zitronensäure, Apfelsäure, Benzoesäure, Zimtsäure, Ascorbinsäure, Methansulfonsäure.

Gegenstand der vorliegenden Erfindung ist die Verwendung der genannten Verbindungen für die Herstellung von Arzneimitteln zur Behandlung chronisch inflammatorischer Prozesse, der Colitis Ulcerosa und des Morbus Crohn und

von Arzneimitteln mit antiproliferativer Wirkung. Die Wirkung der Verbindungen kann durch ihre Hemmung der unselektiven Kationenkanäle (UKK) erklärt werden.

Der Pathophysiologie des chronischen Bronchialasthma liegen entzündliche Prozesse zugrunde, die durch die Aktivierung inflammatorischer Zellen vermittelt werden. (BARHES, 1987; SEIFERT und SCHULTZ, 1991).

Die rezeptor-regulierte Aktivierung inflammatorischer Zellen (z.B. neutrophile Granulozyten und Mastzellen bzw. deren permanente Zellinien HL-60 Zellen oder sensibilisierte, d.h. Gammaglobulin E beladene RBL-Zellen) wird unabhängig von der Art der stimulierenden Agonisten (z.B. Endothelin, PAF, Leukotriene, chemotaktisches Peptid fMLP oder Antigen gegen sensibilisierte Mastzellen) durch Blocker unselektiver Kationenkanäle (UKK) inhibiert (RINK, 1990). Durch diese Kanäle gelangt extrazelluläres Calcium in die Zellen, das für die Persistenz der rezeptor-vermittelten Zellaktivierungen erforderlich ist (PUTNEY, 1990). Wird diese Calciumzufuhr unterbrochen resultiert eine Blockade der Aktivierung inflammatorischer Zellen.

Klassische Calciumantagonisten vom Dihydropyridin- bzw. Phenylalkylamin-Typ hemmen weder UKKs noch inflammatorische Prozesse (WELLS et al., 1986).

Als Maß der Zellaktivierung bzw. als Maß von deren Hemmung durch UKK-Blocker wird fluorometrisch die Kinetik der cytoplasmatischen Calciumionenkonzentration in Fura-2-beladenen Zellen anhand der von GRYNKIEWICZ et al. (1985) beschriebenen Methode quantifiziert. Diese Vorgehensweise hat sich im Rahmen dieser Erfindung als zuverlässige Screeningmethode zur Auffindung von UKK-Blockern erwiesen.

Zur spezifischen Charakterisierung von Blockern der unselektiven Kationenkanäle eignet sich die sogenannte funktionelle THAPSIGARGIN-Inhibition. THAPSIGARGIN ist ein von THASTRUP et al. (Proc. Natl. Acad. Sci. (USA), 87, 2466-2470, 1990) beschriebener Tumorpromotor, der selektiv und irreversibel die $Ca^{2+}$ATPase intrazellulärer, $IP_3$-sensitiver $Ca^{2+}$-Speicher hemmt. Infolge dessen kommt es zu einer raschen Entleerung der $Ca^{2+}$-Speicher. Wie von J. PUTNEY (Calcium, 11, 611-624, 1990) beschrieben stellt die Entleerung dieser Speicher den physiologischen Reiz für die Öffnung unselektiver Kationenkanäle in der Zellmembran dar. Die Folge ist ein massiver Einstrom von $Na^+$ und $Ca^{2+}$ in die Zelle. Aufgrund dieser Eigenschaften eignet sich Thapsigargin als indirekter Stimulator zur agonisten- und $IP_3$-unabhängigen Öffnung der unselektiven Kationenkanäle.

Im Rahmen der vorliegenden Erfindung wurde die Thapsigargin-Stimulation unselektiver Kationenkanäle an HL 60 Zellen (menschliche Leukämiezelle), an hippocamplen und corticalen Neuronenzellen sowie an RBL-Zellen (rat basophilic lymphoma cells) erfolgreich durchgeführt und somit wurde die Existenz dieser Kanäle in den jeweiligen Zellinien nachgewiesen.

Die zytoplasmatische $Ca^{2+}$Konzentration ($[Ca^{2+}]_i$) spielt eine wichtige Rolle bei der Zellproliferation und beim Tumorwachstum (Übersicht siehe L.R. ZACHARSKI, Journal of Medicine 19: 145-177, 1988). Insbesondere der durch Rezeptoraktivierung mit konsekutiver Inositoltrisphosphat-($IP_3$-)-Vermittlung stimulierte $Ca^{2+}$-Einstrom in die Zelle soll von entscheidender Bedeutung sein für die oncogene Zellproliferation (U. KIKKAWA und Y. NISHIZUKA, Ann. REV. CELL. BIOL. 2: 149-178, 1986). Dieser Mechanismus spielt auch eine Rolle bei der Metastasenbildung und der "Multi-Drug-Resistance". (Übersicht siehe die obengenannten Veröffentlichung von L.R. ZACHARSKI.) J. MED. 19: 145-177, 1980.

Diese Hypothese wird dadurch gestützt, daß Thapsigargin als indirekter Stimulator der unselektiven Kationenkanäle (UKK) sowohl zu einem $Ca^{2+}$-overload in der Zelle führt als auch ein hochwirksamer Tumorpromotor ist. (V. THASTRUP et al. Proceedings of the NATL. Acad. Sci: (USA) 87: 2466-2470, 1990.)

Die Blockade des $Ca^{2+}$-Einstroms durch die UKK führt zu einer Normalisierung der intrazellulären Ca-Ionenkonzentration und somit zu einer Hemmung des Tumorwachstums etc.

Klassiche Calciumantagonisten hemmen diese UKK nicht. Überraschend ist festgestellt worden, daß die Verbindungen dieser Erfindung den Calciumeinstrom in die Zelle durch die UKK hemmen.

Wie von S. H. MURCH et al. (Lancet 339 : 381-385, 15. Febr. 1992) gezeigt wurde, spielt Endothelin I eine wichtige pathophysiologische Rolle bei entzündlichen Darmerkrankungen wie der Colitis ulcerosa und des Morbus Crohn. Mit Hilfe immunhistochenischer Methoden konnte festgestellt werden, daß Patienten mit M. Crohn im Bereich der Submucosa und Patienten mit Colitis ulcerosa im Bereich der Lamina propria des Dickdarmepithels signifikant und stark erhöhte Endothelin I Konzentrationen im Vergleich zu gesunden Normalpersonen aufweisen. Es wird angenommen, daß die lokale Ausschüttung von Endothelin massive Vasospasmen mit konsekutiver disseminierter Ischämie mit Mikroinfarkten hervorruft, die als eigentliche Ursache der genannten Erkrankungen angesehen werden. Die vasospasmogene Effektivität des Endothelins wird über einen $Ca^{2+}$-overload vaskulärer Myozyten erklärt. Dabei löst Endothelin primär eine $IP_3$-vermittelte intrazelluläre $Ca^{2+}$-Freisetzung aus, an die sich ein massiver transmembranärer $Ca^{2+}$-Einstrom durch dihydropyridin-insensitive Kanäle anschließt. (M. S. Simonson et al. Clin. Invest. Med. 14: 499-507, 1991; T. Masakai, J. Cardiovasc. Pharmacol. 13:Suppl. 5, S1-S4, 1989; D. W. Hay, R. J. Pharmacol. 100: 383-392, 1990) Bei diesen Kanälen handelt es sich um unselektive Kationen Kanäle, deren Existenz kürzlich auch in Zellen der Dickdarmmukosa beschrieben wurde. (Chr. Siemer und H. Gögelein, Europ. J. Physiol. 420: 319-328, 1992).

Als geeignetes Screening Modell zur Auffindung funktioneller Endothelinantagonisten hat sich die Endothelin stimulierte Aktivierung Fura-2 beladener menschlicher Leukämiezellen (HL 60 Zelle) bewährt. In Anlehnung an G. GRYNKIEWICZ et al. (J. Biol. Chem. 260:340-3450, 1985)läßt sich die intrazelluläre $Ca^{2+}$-Konzentration im Cytoplasma von

HL 60 Zellen (Suspensionen) spektrofluorometrisch verfolgen und als Maß der Zellaktivierung durch Endothelin quantifizieren. Die Stimulation erfolgte durch Zusatz von 0.1 µM Endothelin, sie ließ sich dosisabhängig durch die erfindungsgemäßen Substanzen inhibieren.

Der funktionelle Endothelinantagonismus der erfindungsgemäßen Substanzen wird über eine Blockade der unselektiven Kationen Kanäle vermittelt. Deshalb eignet sich auch der Nachweis eines funktionellen Thapsigargin-Antagonismus an RBL-hm1-Zellen als Screening Methode für funktionelle Endothelinantagonisten.

Ausführung der Untersuchung:

Für Screeningzwecke werden in $Ca^{2+}$ freiem Inkubationsmedium Fura-2-beladene adhäsive RBL-hm 1-Zellen mit 0,1 µM Thapsigargin stimuliert. Nach 4 Minuten wird extrazelluläres $Ca^{2+}$ auf 1,5 mM restituiert und anhand der Fura-2-Fluoreszenz der exzessive Anstieg der cytoplasmatischen $Ca^{2+}$-Konzentration infolge eines massiven transmembranären $Ca^{2+}$-Einstroms durch unselektive Kationenkanäle registriert.

Dieser Einstrom ist ausschließlich und dosisabhängig zu inhibieren durch unselektive Kationen-Kanal-Blocker. Weder klassische Kalziumantagonisten noch spezifische Blocker von Agonisten, die den $IP_3$-Turnover stimulieren können den durch Thapsigargin indirekt ausgelösten transmembranären $Ca^{2+}$-Einstrom hemmen. Die Verbindungen der vorliegenden Erfindung zeichnen sich durch eine Hemmung der UKK aus.

Die fluorometrische Calciummessung im Cytoplasma einzelner adhärenter RBL-hm1-Zellen erfolgt in Analogie zu der von KUDO und OGURA (1986) für neuronale Zellen beschriebenen Methode. Verwendet wird ein AXIOVERT 35 Fluoreszenzmikroskop von ZEISS in Kombination mit einem Imaging-System von HAMAMATSU, bestehend aus dem ICMS-Bildverarbeitungssystem, Restlichtkamera mit Kontrolleinheit und Bildverstärker DVS 3000.

Die Kinetik der cytoplasmatischen $Ca^{+2}$-Konzentration wird als Konzentrations-Zeit-Kurve nach der durch Thapsigargin (0,1 µM) stimulierten Zellaktivierung fortlaufend aufgezeichnet. Verglichen werden die Kurven zweier aktivierter Zellkulturen in Gegenwart und Abwesenheit von 10 µM Testsubstanz. Die Fläche unter diesen Kurven (area under the curve = AUC) wird integriert und als Maß der Zellaktivierung registriert. Die inhibitorische Wirkungsstärke der getesteten UKK-Blocker wird ermittelt anhand folgender Beziehung:

$$\%H = 100 - \frac{AUC_{inh} \times 100}{AUC_{(Kontrolle)}}$$

%H = die prozentuale Hemmung des Calciumeinstroms durch unselektive Kationenkanäle der stimuliert und durch 10 µM Testsubstanz inhibiert wird.

$AUC_{inh}$ = Fläche unter der Kurve, die in Gegenwart des Stimulans plus 10 µM inhibitorischer Testsubstanz aufgezeichnet wird.

AUC Kontr. = Fläche unter der Kurve, die nur nach Zusatz des Stimulans registriert wird.

Literatur zu den obigen Erläuterungen:

BARNES P.J., I.W. RODGER und N.C. THOMSON
Pathogenesis of asthma, in "ASTHMA, basic mechanisms and clinical management" ED by P.J. BARNES; ACADEMIC PRESS, LONDON, 1988

GRYNKIEWICZ G., M. POENIE und R.Y. TSIEN
A new generation of $Ca^{2+}$-indicators with greatly improved fluorescence properties J. BIOL. CHEM. 260: 3440-3450, 1985

HIDE, M. und M.A. BEAVEN
Calcium influx in a rat mast cell (RBL-2H3) line J. BIOL. CHEM. 266 15221-15229, 1991

KUDO, Y. und A. OGURA
Glutamate-induced increase in intracellular $Ca^{2+}$-concentration in isolated hippocampal neurones BR. J. PHARMACOL. 89: 191-198, 1986

PUTNEY, J.W., jr.
Capacitative Calcium entry revised CELL CALCIUM 11: 611-624, 1990

RINK, T.J.
Receptor-mediated calcium entry FEBS LETT. 268: 381-385, 1990

SEIFERT, R. und G. SCHULTZ
The superoxide forming NADPH oxidase of phagocytes: An enzym system regulated by multiple mechanism REV. PHYSIOL. BIOCHEM. PHARMACOL., Vol. 117, SPRINGER VERL., 1991

WELLS, E., C.G. JACKSON, S.T. HARPER, J. MANN and R.P. EAOY
Characterization of primate bronchoalveolar mast cells II, inhibition of histamine, $LTC_4$ and $PGF_{2\alpha}$ release from primate bronchoalveolar mast cells and a comparison with rat peritoneal mast cells
J. IMMUNOL. 137: 3941-3945, 1986.

Meßergebnisse:

Angegeben wird die prozentuale Hemmung der UKK nach Thapsigarginstimulation (0,1 µM Thapsigargin) in RBL-hm 1 Zellen. Die einheitliche Konzentration der Testsubstanzen ist $10^{-5}$ Mol).

RBL - hm 1 Zellen - Thapsigargin (0,1 µM)-Stimulation

Verbindung A %H: 44.59

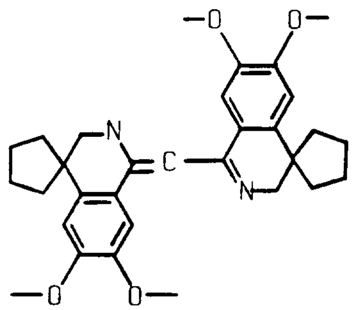

Verbindung B %H: 75.75

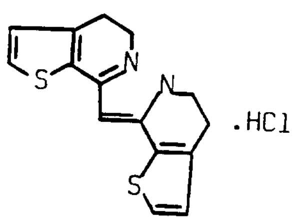

Verbindung C %H: 73.01

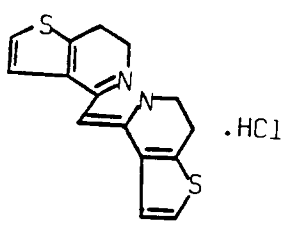

Verbindung D $IC_{50}$: 9.1.10$^{-7}$M

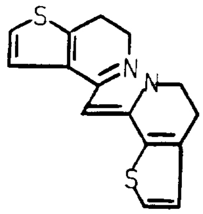

.HCl

Anhand des folgenden Tests kann die funktionelle antiinflammatorische Effektivität gezeigt:

Verwendet werden einzelne an Glasplättchen adhärente RBL-2H3-Zellen (eine den Mastzellen verwandte Tumorzellinie).

Die Kultivierung und Anhaftung der RBL-2H3-Zellen erfolgt in der HIDE und BEAVEN (1991) beschriebenen Methode. Zur Sensibilisierung der adhäsiven RBL-2H3-Zellen werden die Zellen 2 Stunden bei Raumtemperatur mit einer 1:2000 verdünnten käuflichen Gammaglobulin E-Lösung gegen einen Dinitrophenol-Rinderserumalbumin-Komplex (DNP-BSA-Antigen) inkubiert. Anschließend werden die Zellen gewaschen. Durch Zusatz von 0,1 ml DNP-BSA-Lösung (10 µg/ml) erfolgt eine massive immunologische Zellaktivierung, die durch einen cytoplasmatischen $Ca^{2+}$-overload vermittelt wird. Die fluorometrische Calciummessung im Cytoplasma einzelner adhärenter RBL-2H3-Zellen erfolgt in Analogie zu der von KUDO und OGURA (1986) für neuronale Zellen beschriebenen Methode, die auch weiter oben in dieser Beschreibung erläutert wird.

Als Vergleichssubstanz dient in diesen Untersuchungen (10 µM) Chromoglycat, das eine ca. 50 %ige Hemmung der antigen-induzierten Zellaktivierung bewirkt.

In diesem Test zeigen die oben genannten Verbindungen %H Werte, die den oben angegebenen Werten vergleichbar sind.

Untersuchungen an Mikrokulturen verschiedener menschlicher Tumorzellinien mit Hilfe des Tetrazolium Assays zur Feststellung des antiproliferativen Effektes der erfindungsgemäßen Substanzen zeigte sich überraschenderweise, daß die geprüfte Verbindung 5 bis 100 mal wirkungsstärker als die Vergleichssubstanz Verapamil sind.

Die antiproliferative Effektivität der Testsubstanzen wurde mit Hilfe des von MOSMANN (J. IMMUNOL. METH. 65: 55-63, 1983), DENIZOT et al. (J. IMMUNOL. METH. 89: 271-277, 1986) und von J. ELIASON et al. (INT. J. CANCER 46: 113-117, 1990) beschriebenen MTT-Tests bestimmt. (MTT = [3-(4,5-dimethylthiazol-2yl)2,5-diphenyl-tetrazoliumbromid] von CHEMICON Inc. El Segundo, Ca, USA). Dieser Indikator wird nur von lebenden Zellen mit intakten Mitochondrien zu einem blauen Formazan Produkt metabolisiert. Folgende menschliche Tumorzellinien wurden in unserem Test verwendet: A 549 (Adenocarcinom der Lunge), A 431 (Epidermiscarcinom der Vulva), PC 3 (Adenocarcinom der Prostata), SK BR 3 (Adenocarcinom der Mamma), HT 29 (CX1 1) (Adenocarcinom des Colon) und K 562 (chronisch-myeloische Leukämiezelle).

Der Test wurde auf Mikrotiterplatten durchgeführt. Jedes Well enthielt 100 µl einer Zellsuspension (0,2 x $10^6$ Zellen/ml). Als Inkubationsmedium diente RPMI 1640 mit 10% Hitze-inaktiviertem fetalen Kälberserum und 50 µg/ml Gentamycin. Die Zellsuspensionen wurden 0, 24, 48 oder 72 Stunden bei gesättigter Luftfeuchtigkeit in einem $CO_2$ (5%) - Luft (95%)-Gemisch bei 37°C inkubiert in Gegenwart und Abwesenheit von variablen Konzentrationen antiproliferativer Testsubstanzen. Die Testsubstanzen wurden in DMSO (Endverdünnung: 0,1 %) gelöst. Anschließend wurden 10 µl MTT-Lösung (3 mg/ml) und nach 3 Stunden 100 µl einer 0,08 N HCl enthaltenden Isopropanollösung zugesetzt. Nach einer weiteren Stunde wurde die Lichtabsorption bei 570 nm (Vergleichswellenlänge 630 nm) in einem Mikroplattenleser ermittelt. Die Lichtabsorption ist direkt proportional zur Anzahl lebender Zellen. Die halbmaximalen Hemmkonzentrationen der untersuchten Substanzen lagen bei 1 µg/ml.

Die vasospasmolytische Effektivität der obengenannten funktionellen Endothelin- bzw. Thapsigargin-Antagonisten wurde am isolierten Gefäßpräparat bestätigt: An retrograd perfundierten spontan schlagenden LANGENDORFF Herzen aus Ratten wurde die coronare Perfusion fortlaufend mittels elektromagnetischer Flußmessung (Apparatur von Hugo Sachs Elektronik, MARCH), quantifiziert. Mit dieser Meßanordnung lassen sich Ausmaß, Dauer und Verlaufsform von Gefäßspasmen mit großer Genauigkeit registrieren. Perfundiert man mit 100 nM Endothelinkonzentration, so wird der coronare Perfusionsfluß von 11 auf 5 ml/min reduziert. Die Perfusionseinschränkung kann durch die erfindungsgemäßen Substanzen aufgehoben werden. Die Wirkungsstärken der erfindungsgemäßen Verbindungen bezüglich der Thapsigargininhibition an Fura-2-beladenen RBL-hm1-Zellen bzw. der Effektivität der Endothelininhibition an Fura-2 beladenen HL 60 Zellen korrelierte eindeutig mit der am Langendorffpräparat nachgewiesenen vasospasmolytischen Effektivität der untersuchten Substanzen. Es kann daraus geschlossen werden, daß dem vasospasmolytischen Endothelinantagonismus der untersuchten Substanzen eine Blockade der unselektiven Kationen Kanäle unterliegt.

Die Verbindungen können sowohl enteral als auch parenteral verabreicht werden. Als Dosis für die orale Anwendung werden 0,1 bis 500 mg Wirkstoff pro Dosis, für die i.v.-Anwendung von 0,05 bis 150 mg pro Dosis vorgeschlagen. Der gewünschte therapeutische Dosis ist von der Indikation und Darreichungsform abhängig und kann experimentell bestimmt werden.

Beispiele wirksamer Verbindungen werden in den folgenden Tabellen zusammengefaßt:

Tabelle 1

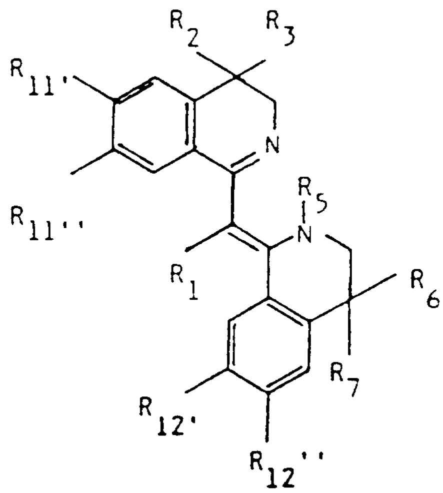

| $R_{11}'$ | $R_{11}''$ | $R_1$ | $R_2$ | $R_3$ | $R_5$ | $R_{12}'$ | $R_{12}''$ | $R_6$ | $R_7$ | Fp°C/Salzform |
|---|---|---|---|---|---|---|---|---|---|---|
| $CH_3O$ | $CH_3O$ | H | $-(CH_2)_4-$ | | H | $CH_3O$ | $CH_3O$ | $-(CH_2)_4-$ | | 119/BS |
| $CH_3SO_2NH$ | $CH_3O$ | H | H | H | H | $CH_3O$ | $CH_3SO_2NH$ | H | H | 233-238 (Z)/BS |
| $CH_3O$ | $CH_3O$ | $n-C_4H_9$ | H | H | $CH_3$ | $CH_3O$ | $CH_3O$ | H | H | 136-137/Cl |
| $CH_3O$ | $CH_3O$ | $n-C_4H_9$ | H | H | H | $CH_3O$ | $CH_3O$ | H | H | 158-159/Cl |
| $CH_3O$ | $CH_3O$ | $CH_3$ | H | H | H | $CH_3O$ | $CH_3O$ | H | H | 180/Cl |
| $CH_3O$ | $CH_3O$ | $CH_3$ | H | H | $CH_3$ | $CH_3O$ | $CH_3O$ | H | H | 177/BS |
| $CH_3O$ | $CH_3O$ | H | H | H | H | $CH_3SO_2O$ | HO | H | H | 182-186/BS |
| $CH_3O$ | $CH_3O$ | H | H | H | $CH_3$ | $CH_3O$ | $CH_3O$ | H | H | 138-140/BS |
| $CH_3O$ | $CH_3SO$, | H | H | H | $CH_3$ | HO | $CH_3O$ | H | H | 222-225/BS |
| $CH_3O$ | $CH_3O$ | $CH_3$ | H | H | $CH_2OH$ | $CH_3O$ | $CH_3O$ | H | H | 155/Cl |
| $CH_3O$ | $CH_3O$ | $n-C_5H_{11}$ | H | H | $CH_3$ | $CH_3O$ | $CH_3O$ | H | H | 75-79(amorph)/BS |
| $CH_3O$ | $CH_3O$ | $C_2H_5$ | H | H | $CH_3$ | $CH_3O$ | $CH_3O$ | H | H | 80 (amorph)/BS |
| $CH_3O$ | $CH_3O$ | $C_6H_5$ | H | H | H | $CH_3O$ | $CH_3O$ | H | H | 95-100/BS |
| $CH_3O$ | $CH_3O$ | $CH_3$ | H | H | H | $-O-CH_2-O-$ | | H | H | 156-158/Cl |
| $CH_3O$ | $CH_3O$ | $C_2H_5$ | H | H | H | $-O-CH_2-O-$ | | H | H | 148-155/Cl |
| $CH_3O$ | $CH_3O$ | $C_2H_5$ | H | H | H | $CH_3O$ | $CH_3O$ | H | H | 177-179/Cl |
| $CH_3O$ | $CH_3O$ | H | H | H | H | $-O-CH_2-O-$ | | H | H | 251-253/Cl |
| $-O-CH_2-O-$ | | H | H | H | H | $-O-CH_2-O-$ | | H | H | 251-253(Z)/Cl |
| $CH_3O$ | $CH_3O$ | $CH_3CONH$ | H | H | H | $CH_3O$ | $CH_3O$ | H | H | 171-172/Cl |
| $-O-CH_2-O-$ | | $C_6H_5$ | H | H | H | $-O-CH_2-O-$ | | H | H | 191-195/BS |

BS = Base
Cl = Chlorid
Z = Zersetzung

Tabelle 2

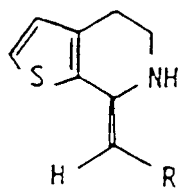

| R | Salzform | Fp(°C) |
|---|---|---|
| 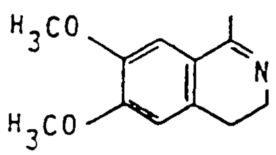 | Cl | 218-222 |
| 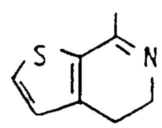 | Cl | 239-235 |

Tabelle 3

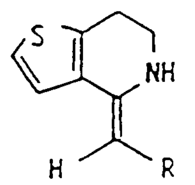

| R | Salzform | Fp(°C) |
|---|---|---|
| 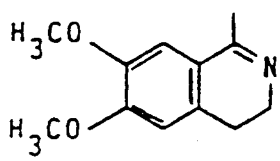 | Cl | 162-172 |
| 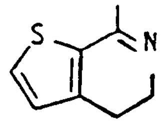 | Cl | 237-239 |
| 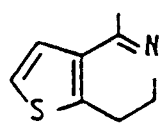 | Cl | 250-253 |

Pharmazeutische Anwendungsbeispiele

| a) Dragees<br>1 Drageekern enthält: | |
|---|---|
| Wirkstoff der allgemeinen Formel I | 30,0 mg |
| Milchzucker | 100,0 mg |
| Maisstärke | 75,0 mg |
| Gelatine | 3,0 mg |
| Magnesiumstearat | 2,0 mg |
| | 210,0 mg |

Herstellung

Die Mischung der Wirksubstanz mit Milchzucker und Maisstärke wird mit einer 10%-igen wässerigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässerigen Suspension von Zucker, Titandioxyd, Talkum und Gummi arabicum aufgebracht wird. Die fertigen Dragees werden mit Bienenwachs poliert.

| b) Tabletten | |
|---|---|
| Wirkstoff der allgemeinen Formel I | 30,0 mg |
| Milchzucker | 100,0 mg |
| Maisstärke | 70,0 mg |
| löslich Stärke | 7,0 mg |
| Magnesiumstearat | 3,0 mg |
| | 210,0 mg |

Herstellung

Wirkstoff und Magnesiumstearat werden mit einer wässerigen Lösung der löslichen Stärke granuliert, das Granulat getrocknet und innig mit Milchzucker und Maisstärke vermischt. Das Gemisch wird sodann zu Tabletten von 210 mg Gewicht verpreßt.

| c) Kapseln | |
|---|---|
| Wirkstoff gemäß Anspruch 1 | 20,0 mg |
| Milchzucker | 230,0 mg |
| Maisstärke | 40,0 mg |
| Talk | 10,0 mg |
| | 300,0 mg |

Herstellung

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

## Patentansprüche

1. Verwendung einer Verbindung der allgemeinen Formel I

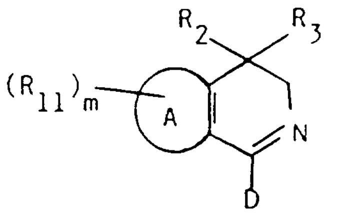

I

worin

A einen Benzo- oder Thienorest bedeutet;

$R_2$ und $R_3$ unabhängig voneinander Wasserstoff oder $(C_1\text{-}C_5)$Alkyl oder gemeinsam mit dem Kohlenstoffatom, an das sie gebunden sind, einen 5- oder 6-gliedrigen Carbocyclus bedeuten;

$R_{11}$ $(C_1\text{-}C_4)$Alkyl, Halogen (F, Cl, Br, J), Hydroxy, $(C_1\text{-}C_4)$Alkoxy, Amino, Thiomethyl, Methansulfonyloxy oder Methansulfonamido bedeutet, oder zwei benachbarte Substituenten $R_{11}$ zusammen $-O-CH_2-O-$ oder $-O-CH_2-CH_2-O-$ sind;

m 0, 1, 2 oder 3 bedeutet, wenn A ein Benzorest ist, und 0, 1 oder 2 bedeutet, wenn A ein Thienorest ist;

und D eine Gruppe der Formel Ia bedeutet

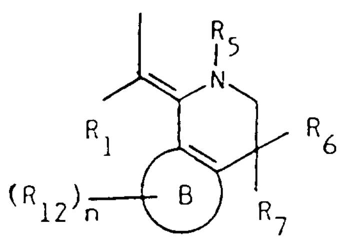

Ia

wobei in der Gruppe der Formel Ia

B einen Benzo- oder Thienorest bedeutet;

$R_1$ Wasserstoff, $(C_1\text{-}C_{10})$Alkyl, Phenyl, Phenyl$(C_1\text{-}C_5)$alkyl, $(C_1\text{-}C_4)$Alkoxy oder -NHCOX (worin X $(C_1\text{-}C_5)$Alkyl ist) bedeutet;

$R_5$ Wasserstoff, $(C_1\text{-}C_4)$Alkyl oder Hydroxymethyl bedeutet;

$R_6$ und $R_7$ unabhängig voneinander Wasserstoff oder $(C_1\text{-}C_5)$Alkyl oder gemeinsam mit dem Kohlenstoffatom, an das sie gebunden sind, einen 5- oder 6-gliedrigen Carbocylcus bedeuten;

$R_{12}$ $(C_1\text{-}C_4)$Alkyl, Halogen (F, Cl, Br, J), Hydroxy, $(C_1\text{-}C_4)$Alkoxy, Amino, Thiomethyl, Methansulfonyloxy oder Methansulfonamido bedeutet, oder zwei benachbarte Substituenten $R_{12}$ zusammen $-O-CH_2-O-$ oder $-O-CH_2-CH_2-O-$sind;

und n 0, 1, 2 oder 3 bedeutet, wenn B ein Benzorest ist, und 0, 1 oder 2 bedeutet, wenn B ein Thienorest ist;

oder deren pharmazeutisch annehmbaren Salzes mit einer anorganischen oder organischen Säure für die Herstellung eines Arzneimittels für die Behandlung chronisch inflammatorischer Prozesse, der Colitis Ulcerosa

und des Morbus Crohn und eines Arzneimittels mit antiproliferativer Wirkung.

**2.** Verwendung einer Verbindung nach Anspruch 1, worin

$R_1$ Wasserstoff, $(C_1-C_{10})$Alkyl, Phenyl$(C_1-C_5)$alkyl oder -NHCOX (worin X $(C_1-C_5)$Alkyl ist) bedeutet;

$R_{11}$ und $R_{12}$ unabhängig voneinander $(C_1-C_4)$Alkyl, Hydroxy, $(C_1-C_4)$Alkoxy, Methansulfonyloxy oder Methansulfonamido bedeuten, oder zwei benachbarte Substituenten $R_{11}$ beziehungsweise $R_{12}$ zusammen $-O-CH_2-O-$ oder $-O-CH_2-CH_2-O-$ sind.

worin vorzugsweise $R_1$ Wasserstoff, $(C_1-C_{10})$Alkyl oder -NHCOX (worin X $(C_1-C_5)$Alkyl ist) bedeutet;

$R_2$, $R_3$, $R_6$ und $R_7$ unabhängig voneinander Wasserstoff bedeuten oder $R_2$ mit $R_3$ und/oder $R_6$ mit $R_7$ und dem jeweiligen Kohlenstoffatom, an das sie gebunden sind, einen 5- oder 6-gliedrigen Carbocyclus bedeuten;

$R_{11}$ und $R_{12}$ unabhängig voneinander Hydroxy, $(C_1-C_4)$Alkoxy, Methansulfonyloxy oder Methansulfonamido bedeuten, oder zwei benachbarte Substituenten $R_{11}$ beziehungsweise $R_{12}$ zusammen $-O-CH_2-O-$ oder $-O-CH_2-CH_2-O-$ sind; worin insbesondere $R_1$ Wasserstoff, $(C_1-C_6)$Alkyl, oder $-NHCOCH_3$ bedeutet und/oder

$R_5$ Wasserstoff, Methyl oder Hydroxymethyl bedeutet und/oder

$R_2$, $R_3$, $R_6$ und $R_7$ unabhängig voneinander Wasserstoff bedeuten oder $R_2$ mit $R_3$ und/oder $R_6$ mit $R_7$ und dem jeweiligen Kohlenstoffatom, an das sie gebunden sind, einen 5-gliedrigen Carbocyclus bedeuten und/oder

$R_{11}$ und $R_{12}$ unabhängig voneinander Hydroxy, Methoxy, Methansulfonoxy oder Methansulfonamido bedeuten, oder zwei benachbarte Substituenten $R_{11}$ beziehungsweise $R_{12}$ zusammen $-O-CH_2-O-$ sind.

**3.** Verwendung einer Verbindung nach Anspruch 1 oder 2, worin $R_1$ und $R_5$ Wasserstoff sind.

**4.** Verwendung einer Verbindung nach einem der Ansprüche 1 bis 3, worin, wenn $R_2$, $R_3$, $R_6$ und $R_7$ Wasserstoff sind.

**5.** Verwendung einer Verbindung nach einem der Ansprüche 1 bis 3, worin $R_2$ und $R_3$ beziehungsweise $R_6$ und $R_7$ je zusammen $-(CH_2)_4-$ sind.

**6.** Verwendung einer Verbindung nach einem der Ansprüche 1 bis 5, worin A und B unabhängig voneinander je eine Thienogruppe sind, vorzugsweise 2,3-Thieno.

**7.** Verwendung einer Verbindung nach Anspruch 6, worin A und B eine unsubstituierte Thienogruppe sind.

**8.** Verwendung einer Verbindung nach einem der Ansprüche 1 bis 5, worin A und/oder B ein Benzorest ist, worin m beziehungsweise n 2 bedeuten.

**9.** Verwendung einer Verbindung nach Anspruch 8, worin, wenn A und/oder B ein Benzorest ist, die beiden Substituenten $R_{11}$ und/oder $R_{12}$ im Benzorest in meta- beziehungsweise para-Position zu den Fusionspunkten des Restes A beziehungsweise B stehen.

**10.** Verwendung einer Verbindung nach einem der Ansprüche 1 bis 5, 8 und 9, worin $R_{11}$ und $R_{12}$ unabhängig voneinander Hydroxy, $(C_1-C_4)$Alkoxy (vorzugsweise Methoxy) oder die 2 benachbarten Substituenten $R_{11}$ beziehungsweise $R_{12}$ zusammen $-O-CH_2-O-$ sind.

**11.** Verwendung einer Verbindung nach Anspruch 1, die

Verbindung A

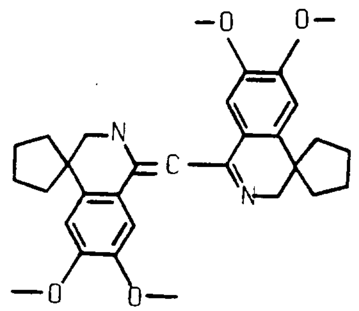

Verbindung B

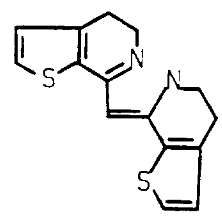

Verbindung C

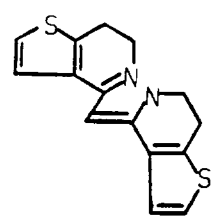

Verbindung D

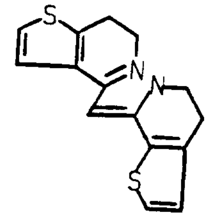

oder ihr physiologisch unbedenkliches Salz ist.

**Claims**

1. Use of a compound of general formula I

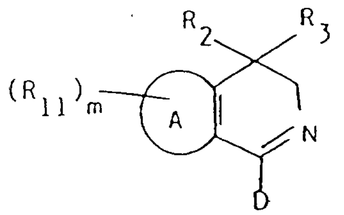

I

wherein

A denotes a benzo- or thieno- group;

$R_2$ and $R_3$ independently of each other denote hydrogen or $(C_{1-5})$alkyl or, together with the carbon atom to which they are bound, a 5- or 6-membered carbocyclic group;

$R_{11}$ denotes $(C_{1-4})$alkyl, halogen (F, Cl, Br, I), hydroxy, $(C_{1-4})$alkoxy, amino, thiomethyl, methanesulphonyloxy or methanesulphonamido, or two adjacent $R_{11}$ substituents together denote $-O-CH_2-O-$ or $-O-CH_2-CH_2-O-$;

m denotes 0, 1, 2 or 3, if A is a benzo group, and 0, 1 or 2, if A is a thieno group;

and D denotes a group of formula Ia

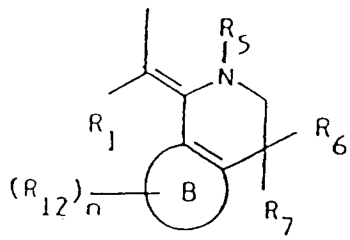

Ia

whilst in the group of formula Ia

B denotes a benzo- or thieno- group;

$R_1$ denotes hydrogen, $(C_{1-10})$alkyl, phenyl, phenyl $(C_{1-5})$ alkyl, $(C_{1-4})$ alkoxy or -NHCOX (wherein X is $(C_{1-5})$alkyl);

$R_5$ denotes hydrogen, $(C_{1-4})$alkyl or hydroxymethyl;

$R_6$ and $R_7$ independently of each other denote hydrogen or $(C_{1-5})$alkyl or together with the carbon atom to which they are bound denote a 5- or 6-membered carbocyclic group;

$R_{12}$ denotes $(C_{1-4})$alkyl, halogen (F, Cl, Br, I), hydroxy, $(C_{1-4})$alkoxy, amino, thiomethyl, methanesulphonyloxy or methanesulphonamido, or two adjacent $R_{12}$ substituents together denote $-O-CH_2-O-$ or $-O-CH_2-CH_2-O-$;

and n denotes 0, 1, 2 or 3 if B is a benzo group and 0, 1 or 2 if B is a thieno group;
or the pharmaceutically acceptable salt thereof with an inorganic or organic acid for the preparation of a pharmaceutical composition for treating chronic inflammatory processes, ulcerative colitis and Crohn's disease and for producing a pharmaceutical composition having an antiproliferative effect.

**2.** Use of a compound according to claim 1, wherein

$R_1$ denotes hydrogen, $(C_{1-10})$alkyl, phenyl$(C_{1-5})$alkyl or -NHCOX (wherein X is $(C_{1-5})$alkyl);

$R_{11}$ and $R_{12}$ independently of each other denote $(C_{1-4})$alkyl, hydroxy, $(C_{1-4})$alkoxy, methanesulphonyloxy or methanesulphonamido, or two adjacent $R_{11}$ and $R_{12}$ substituents together denote $-O-CH_2-O-$ or $-O-CH_2-CH_2-O-$;
wherein preferably $R_1$ denotes hydrogen, $(C_{1-10})$alkyl or -NHCOX (wherein X is $(C_{1-5})$alkyl);

$R_2$, $R_3$, $R_6$ and $R_7$ independently of one another denote hydrogen or $R_2$ together with $R_3$ and/or $R_6$ together with $R_7$ and the associated carbon atom to which they are bound denote a 5- or 6-membered carbocyclic group;

$R_{11}$ and $R_{12}$ independently of each other denote hydroxy, $(C_{1-4})$alkoxy, methanesulphonyloxy or methanesulphonamido, or two adjacent $R_{11}$ and $R_{12}$ substituents together denote $-O-CH_2-O-$ or $-O-CH_2-CH_2-O-$;
wherein in particular $R_1$ denotes hydrogen, $(C_{1-6})$alkyl or $-NHCOCH_3$ and/or

$R_5$ denotes hydrogen, methyl or hydroxymethyl and/or

$R_2$, $R_3$, $R_6$ and $R_7$ independently of one another denote hydrogen or $R_2$ together with $R_3$ and/or $R_6$ together with $R_7$ and the associated carbon atom to which they are bound denote a 5-membered carbocyclic group and/or

$R_{11}$ and $R_{12}$ independently of each other denote hydroxy, methoxy, methanesulphonyloxy or methanesulphonamido, or two adjacent $R_{11}$ and $R_{12}$ substituents together represent $-O-CH_2-O-$.

3. Use of a compound according to claim 1 or 2, wherein $R_1$ and $R_5$ denote hydrogen.

4. Use of a compound according to one of claims 1 to 3, wherein $R_2$, $R_3$, $R_6$ and $R_7$ denote hydrogen.

5. Use of a compound according to one of claims 1 to 3, wherein $R_2$ and $R_3$ or $R_6$ and $R_7$ together represent $-(CH_2)_4-$.

6. Use of a compound according to one of claims 1 to 5, wherein A and B independently of each other denote a thieno group, preferably 2,3-thieno.

7. Use of a compound according to claim 6, wherein A and B denote an unsubstituted thieno group.

8. Use of a compound according to one of claims 1 to 5, wherein A and/or B is a benzo group, wherein m or n denotes 2.

9. Use of a compound according to claim 8, wherein, if A and/or B is a benzo group, the two substituents $R_{11}$ and/or $R_{12}$ in the benzo group are in the meta- or para-position, respectively, to the fusion points of the group A or B.

10. Use of a compound according to one of claims 1 to 5, 8 and 9, wherein $R_{11}$ and $R_{12}$ independently of each other denote hydroxy, $(C_{1-4})$alkoxy (preferably methoxy) or the 2 adjacent $R_{11}$ and $R_{12}$ substituents together denote $-O-CH_2-O-$.

11. Use of a compound according to claim 1 which is

Compound A

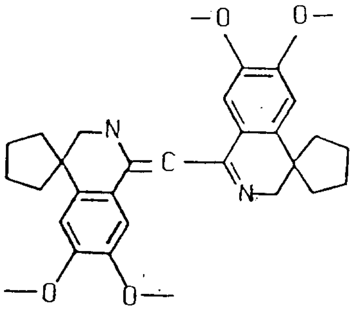

Compound B

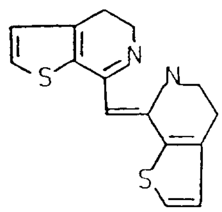

Compound C

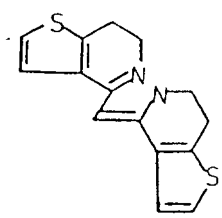

Compound D

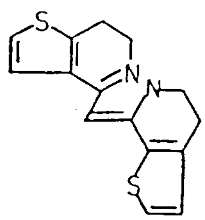

or a physiologically acceptable salt thereof.

**Revendications**

1. Utilisation d'un composé de formule générale I

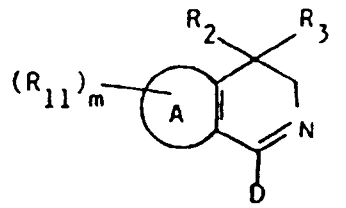

I

dans laquelle

A représente un reste benzo ou thiéno;
$R_2$ et $R_3$ représentent indépendamment l'un de l'autre l'hydrogène ou alkyle en $C_1$-$C_5$ ou avec l'atome de carbone auquel ils sont liés un carbocycle à 5 ou 6 chaînons;
$R_{11}$ représente alkyle en $C_1$-$C_4$, halogène (F, Cl, Br, I), hydroxyle, alcoxy en $C_1$-$C_4$, amino, thiométhyle, méthanesulfonyloxy ou méthanesulfonamido, ou deux substituants $R_{11}$ voisins sont ensemble -O-$CH_2$-O- ou -O-$CH_2$-$CH_2$-O-;
m représente 0, 1, 2 ou 3 lorsque A est un reste benzo et 0, 1 ou 2 lorsque A est un reste thiéno;
et D représente un groupe de formule Ia

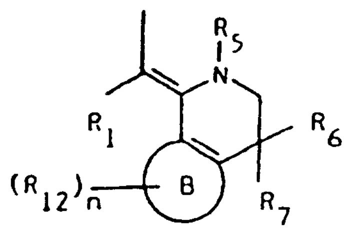

Ia

dans le groupe de formule Ia
B représentant un reste benzo ou thiéno;
$R_1$ représentant l'hydrogène, alkyle en $C_1$-$C_{10}$, phényle, phénylalkyle en $C_1$-$C_5$, alcoxy en $C_1$-$C_4$ ou -NHCOX (où X est alkyle en $C_1$-$C_5$);
$R_5$ représentant l'hydrogène, alkyle en $C_1$-$C_4$ ou hydroxyméthyle;
$R_6$ et $R_7$ représentant indépendamment l'un de l'autre l'hydrogène ou alkyle en $C_1$-$C_5$ ou avec l'atome de carbone auquel ils sont liés un carbocycle à 5 ou 6 chaînons;
$R_{12}$ représentant alkyle en $C_1$-$C_4$, halogène (F, Cl, Br, I), hydroxyle, alcoxy en $C_1$-$C_4$, amino, thiométhyle, méthanesulfonyloxy ou méthanesulfonamido, ou deux substituants $R_{12}$ voisins étant ensemble -O-$CH_2$-O- ou -O-$CH_2$-$CH_2$-O-;
et n représentant 0, 1, 2 ou 3 lorsque B est un reste benzo et 0, 1 ou 2 lorsque B est un reste thiéno;
ou de ses sels pharmaceutiquement acceptables avec un acide inorganique ou organique pour la préparation d'un médicament pour le traitement des processus inflammatoires chroniques, de la colite ulcéreuse et de la maladie de Crohn et d'un médicament à effet antiprolifératif.

2. Utilisation d'un composé selon la revendication 1 dans lequel $R_1$ représente l'hydrogène, alkyle en $C_1$-$C_{10}$, phénylalkyle en $C_1$-$C_5$ ou -NHCOX (où X est alkyle en $C_1$-$C_5$);

$R_{11}$ et $R_{12}$ représentent indépendamment l'un de l'autre alkyle en $C_1$-$C_4$, hydroxyle, alcoxy en $C_1$-$C_4$, méthanesulfonyloxy ou méthanesulfonamido, ou deux substituants $R_{11}$ ou $R_{12}$ voisins sont ensemble -O-$CH_2$-O- ou -O-$CH_2$-$CH_2$-O-;
où $R_1$ représente de préférence l'hydrogène, alkyle en $C_1$-$C_{10}$ ou -NHCOX (où X est alkyle en $C_1$-$C_5$);
$R_2$, $R_3$, $R_6$ et $R_7$ représentent indépendamment les uns des autres l'hydrogène ou $R_2$ et $R_3$ et/ou $R_6$ et $R_7$ représentent avec l'atome de carbone auquel ils sont liés un carbocycle à 5 ou 6 chaînons;
$R_{11}$ et $R_{12}$ représentent indépendamment l'un de l'autre hydroxyle, alcoxy en $C_1$-$C_4$, méthanesulfonyloxy ou méthanesulfonamido, ou deux substituants $R_{11}$ ou $R_{12}$ voisins sont ensemble -O-$CH_2$-O- ou -O-$CH_2$-$CH_2$-O-;
où en particulier $R_1$ représente l'hydrogène, alkyle en $C_1$-$C_6$ ou -$NHCOCH_3$ et/ou

$R_5$ représente l'hydrogène, méthyle ou hydroxyméthyle et/ou

$R_2$, $R_3$, $R_6$ et $R_7$ représentent indépendamment les uns des autres l'hydrogène ou $R_2$ et $R_3$ et/ou $R_6$ et $R_7$ représentent avec l'atome de carbone auquel ils sont liés un carbocycle à 5 chaînons et/ou

$R_{11}$ et $R_{12}$ représentent indépendamment l'un de l'autre hydroxyle, méthoxy, méthanesulfonyloxy ou méthanesulfonamido, ou deux substituants $R_{11}$ ou $R_{12}$ voisins sont ensemble -O-$CH_2$-O-.

3. Utilisation d'un composé selon la revendication 1 ou 2 où $R_1$ et $R_5$ sont l'hydrogène.

4. Utilisation d'un composé selon l'une des revendications 1 à 3 où $R_2$, $R_3$, $R_6$ et $R_7$ sont l'hydrogène.

5. Utilisation d'un composé selon l'une des revendications 1 à 3 où $R_2$ et $R_3$ ou $R_6$ et $R_7$ sont ensemble $-(CH_2)_4-$.

6. Utilisation d'un composé selon l'une des revendications 1 à 5 où A et B sont indépendamment l'un de l'autre chacun un groupe thiéno, de préférence 2,3-thiéno.

7. Utilisation d'un composé selon la revendication 6 où A et B sont un groupe thiéno non substitué.

8. Utilisation d'un composé selon l'une des revendications 1 à 5 où A et/ou B est un reste benzo, où m ou n représentent 2.

9. Utilisation d'un composé selon la revendication 8 où, lorsque A et/ou B est reste benzo, les deux substituants $R_{11}$ et/ou $R_{12}$ dans le reste benzo sont situés en position méta ou para par rapport aux points de fusion du reste A ou B.

10. Utilisation d'un composé selon l'une des revendications 1 à 5, 8 et 9 où $R_{11}$ et $R_{12}$ sont indépendamment l'un de l'autre hydroxyle, alcoxy en $C_1$-$C_4$ (de préférence méthoxy) ou les deux substituants $R_{11}$ ou $R_{12}$ voisins sont ensemble -O-$CH_2$-O-.

11. Utilisation d'un composé selon la revendication 1 qui est

le composé A

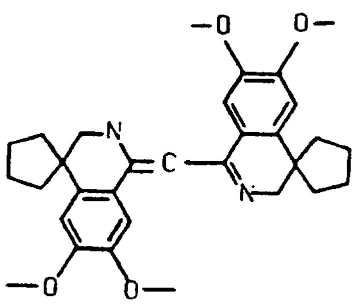

le composé B

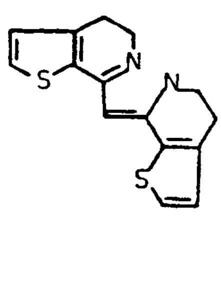

le composé C

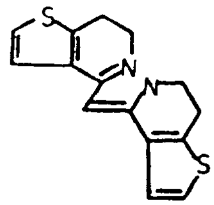

le composé D

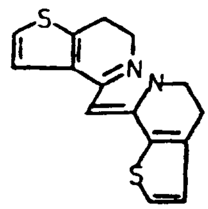

ou son sel physiologiquement acceptable.